**Europäisches Patentamt**

**European Patent Office**

**Office européen des brevets**

(19)

(11) Veröffentlichungsnummer: **0 177 781 B1**

(12) **EUROPÄISCHE PATENTSCHRIFT**

(45) Veröffentlichungstag der Patentschrift: **27.06.90**

(21) Anmeldenummer: **85111403.3**

(22) Anmeldetag: **09.09.85**

(51) Int. Cl.⁵: **A 61 L 25/00, C 08 L 33/06, C 08 K 3/32**

(54) **Knochenzement und Verfahren zu seiner Herstellung.**

(30) Priorität: **10.09.84 PCt/ep84/00275**
**16.07.85 DE 3525362**

(43) Veröffentlichungstag der Anmeldung:
**16.04.86 Patentblatt 86/16**

(45) Bekanntmachung des Hinweises auf die
Patenterteilung:
**27.06.90 Patentblatt 90/26**

(84) Benannte Vertragsstaaten:
**AT BE CH DE FR GB IT LI LU NL SE**

(56) Entgegenhaltungen:
**EP-A-0 018 496**
**DE-A-2 905 878**

(73) Patentinhaber: **Draenert, Klaus,**
**Dr.med.Dr.med.habil.**
**Gabriel-Max-Strasse 3**
**D-8000 München 90 (DE)**

(72) Erfinder: **Draenert, Klaus, Dr.med.Dr.med.habil.**
**Gabriel-Max-Strasse 3**
**D-8000 München 90 (DE)**

(74) Vertreter: **Vossius & Partner**
**Siebertstrasse 4 P.O. Box 86 07 67**
**D-8000 München 86 (DE)**

## Beschreibung

Die Erfindung betrifft einen Knochenzement auf der Basis von Polyacrylsäureester und/oder Polymethacrylsäureester und/oder deren Copolymerisaten, der insbesondere als Knochenersatz-, Knochenverbund- und Prothesenverankerungsmaterial Verwendung finden kann.

Bekannte Knochenzemente bestehen aus einer polymeren Komponente (Acrylsäureester- oder Methacrylsäureester-"Präpolymerisat", das in Form von Kügelchen vorliegt und als "Kugelpolymerisat" oder "Perlpolymerisat" bezeichnet wird), einem monomeren Acryl- oder Methacrylsäurederivat, z.B. Methacrylsäuremethylester, einem Polymerisationskatalysator und gegebenenfalls einem Stabilisator und einem Aktivator oder Beschleuniger. Zum Gebrauch werden die Komponenten homogen vermischt und in geeigneter Weise, z.B. mittels einer Zementspritze an den Applikationsort gebracht. Die monomere Komponente härtet durch Polymerisation und umschließt dabei das Präpolymerisat, so daß ein homogenes Polymergefüge entsteht.

Knochenzemente auf der Basis von Polyacrylsäureester sind nicht resorbierbar, sondern werden durch Einwachsen von körpereigenem Gewebe in dieses eingebaut und fest umschlossen. Eine stabile Verbindung Implantat/Implantatlager ist nur durch eine Art Verblockung möglich. Eine Verbindung auf molekularer Ebene ist bislang weder bewiesen noch gezeigt worden. Unter biomechanisch günstigen Voraussetzungen, d.h. bei Vermeidung von Überlastungen der Grenzzone zwischen Implantat und Knochen, kommt es jedoch zum geschlossenen knöchernen Kontakt und damit zur Verblockung des Implantates im knöchernen Implantatlager des Patienten.

Sehr häufig kommt es jedoch durch biomechanische Überbeanspruchung des Implantates, d.h. durch auf das Implantat wirkende Biege- und Scherkräfte, zur Ausbildung von Oberflächenverschiebungen des Implantat gegen das Implantatlager (Knochenoberfläche), die zur Knochenresorption, den schnellen Knochenumbau von Lamellen in einen minderwertigeren Geflechtknochen und schließlich zur Bildung einer straffen Bindegewebsscheide führen. Aufgrund der Überlastung des Interfaces durch Scher- und Biegebeanspruchung können alle Stadien der Lockerung bis zur vollständigen Auslockerung des gesamten Implantates beobachtet werden.

Es hat nicht an Versuchen gefehlt, die Grenzfläche von Implantat und Knochen durch verbesserte Implantationsmethoden (Impaktoren) zu vergrößern. Insbesondere wurde auch versucht, durch Beimengung resorbierbaren Materials die angrenzende Oberfläche zum Implantatlager erheblich zu vergrößern; vgl. DE—B—29 05 878. Ist durch Beimengung solcher Füller oder Füllsubstanzen die Oberflächenvergrößerung in eindrucksvoller Weise zu verwirklichen und auch das Einwachsen von Knochen bis tief in das Implantat möglich und nachgewiesen worden, so bedeutet die Beimengung von Füllsubstanzen auf der anderen Seite eine Schwächung der mechanischen Festigkeiten dieser Knochenzemente im Hinblick auf die Biegung und Verwringung der Prothesenzementscheide.

Diese Schwächung der mechanischen Festigkeit der Knochenzemente ist vor allem bei den Zementen besonders ausgeprägt, die wässrige Gele oder lösliche Füllstoffe dem Zement beimischen; vgl. DE—A—25 18 153 und J. Biomed. Mater. Res. Bd. 11 (1977), S. 373—394.

Allen diesen Knochenzementen fehlt letztendlich die erforderliche Stabilität, um eine stabile Zementscheide um eine Prothesenkomponente zu bilden.

Dieser Nachteil wurde verschiedentlich mit der Schaffung von Faserzementen zu lösen versucht. So wurde beispielsweise in der DE—A—27 24 814 durch Beimengung von verschieden langen Glasfasern die Biegefestigkeit der Knochenzemente ganz erheblich erhöht. Es zeigte sich jedoch, daß die Beimengung dieser Fasern zu sogenannten "Gitterphänomenen" führte, indem sich die Fasern als Gitter dem Knochenlager anlegten und nicht der Zementmatrix in die quer verankernden Poren des knöchernen Lagers folgten. Dies führte zu einer erheblichen Schwächung des Zementes in den für die Verankerung hauptsächlich notwendigen Querauflagen des Knochens. Auf der anderen Seite nahm die Mischbarkeit und Verarbeitbarkeit der Knochenzemente drastisch ab, so daß letztlich der Ausguß der Knochenhöhlen mit diesen Faserzementen der Primärapplikation von Keramikprothesen gleichkam. Die plastische Querverankerung der Knochenzemente war unzureichend. Auch die in der EP—A—6 414 beschriebene Faserverstärkung mit Kohlefasern zeigte dieselben Ergebnisse und war aus diesem Grunde für den klinischen Einsatz nicht zu gebrauchen.

Der Erfindung liegt die Aufgabe zugrunde, einen Knochenzement der eingangs genannten Art sowie ein Verfahren zu seiner Herstellung bereitzustellen, der sowohl eine große Oberfläche zum Implantatlager schafft, als auch auf der anderen Seite eine genügende mechanische Festigkeit aufweist und eine geschlossene Zementscheide um die Prothesenkomponente bildet.

Diese Aufgabe wird durch den Knochenzement und das Herstellungsverfahren gemäß der Patentansprüche gelöst. Die Erfindung beruht dabei auf dem überraschenden Befund, daß man durch Vergrößerung der Oberfläche des Kugelpolymerisates, beispielsweise durch Ersatz eines Teiles des Kugelpolymerisates durch Acrylsäureester- und/oder Methacrylsäureesterfasern bestimmter Länge und Dicke und/oder durch Zertrümmerung eines Teiles oder des gesamten Kugelpolymerisates und/oder durch chemische Veränderungen seiner Oberfläche, ohne die Mischbarkeit des Zementes wesentlich zu verändern, eine erheblich höhere mechanische Stabilität erreichen kann. Ferner wurde gefunden, daß auch allein die Vereinheitlichung der Größenverteilung der Polymerfraktion bereits eine starke Fertigkeitszunahme bewirkt.

Im Rahmen der Erfindung sollen dabei die Begriffe "Polyacrylsäureester" und "Polymethacrylsäureester" jeweils auch Copolymerisate der genannten Verbindungen umfassen. Als Polyacrylate und Polymethacrylate kommen alle für Knochenzemente geeigneten Polymerisate und Copolymerisate von Acryl- und Methacrylsäureestern in Frage. Bevorzugt sind die Ester mit aliphatischen $C_1$—$C_6$-Alkoholen, insbesondere die Methylester.

Das Verhalten des Perlpolymerisates während der Polymerisation der im Handel befindlichen Knochenzemente hat gezeigt, daß die Kugel zwar morphologisch wertvoller Bestandteil als formgebendes Element für die knochenbildende Zelle ist, daß sie aber für den Matrixverbund des Knochenzementes eine ungünstige, nämlich die kleinste Oberfläche hat. Durch die Vergrößerung dieser Oberfläche durch chemische Vorbehandlung (Lösungsmittel) oder mechanische Zertrümmerung und vieleckige Gestaltung der Kugeltrümmer kann eine ganz erhebliche mechanische Festigkeitssteigerung dieser Knochenzemente erreicht werden.

Das erfindungsgemäß verwendete Polymerpulver (Präpolymerisat-Teilchen) besteht aus Polyacrylsäure oder Polymethacrylsäure oder Polyacrylsäureester oder Polymethacrylsäureester oder Copolymerisaten aus den genannten Verbindungen.

Die Korngröße der Präpolymerisat-Teilchen beträgt vorzugsweise zwischen 5 und 60 µm, besonders bevorzugt 10 bis 45 µm, mit dem Maximum bei etwa 40 µm.

Das mittlere Molekulargewicht der Präpolymerisat-Teilchen beträgt vorzugsweise zwischen 100,000 und 2,000,000.

Um eine möglichst große Oberfläche der Präpolymerisat-Teilchen zu erzielen, wird das Polymerpulver mechanisch und/oder chemisch vorbehandelt.

Bei der mechanischen Vorbehandlung werden die Polymerkugeln vorzugsweise in einer Mühle, z.B. einer Kugelmühle, oder einem Mörser zermahlen oder zerstampft, um mindestens die größeren Polymerkugeln zu zertrümmern. Die Pulvermischung besteht anschließend aus ganzen Polymerkugeln und zertrümmerten Kugelbruchstücken oder ganz aus Kugelbruchstücken.

Bei der chemischen Vorbehandlung wird das Polymerpulver durch anorganische oder organische Lösungsmittel oder Substanzen vorbehandelt und die Oberfläche der Polymerkugeln wird derart angelöst oder vergrößert, daß später eine optimale Haftung zwischen den Polymerkugeln und der auspolymerisierenden Monomer-Flüssigkeit entsteht.

Nach der Behandlung, z.B. in eine Kugelmühle, ist die Größenverteilung der Teilchen eine veränderte Gaußverteilung. Er ist jedoch besonders vorteilhaft, daß die Teilchen eine möglichst einheitliche, konstante Teilchengröße aufweisen. Dadurch wird der Monomerverbrauch geringer.

Die Teilchen lassen sich geometrisch dichter packen, die Packung wird stabiler und die mechanische Festigkeit des Knochenzements wird erhöht.

Besonders bevorzugt ist eine Teilchengröße von etwa 30—40 µm. Ein derartiges "gereinigtes Spektrum" der Teilchen läßt sich z.B. durch Sieben, beispielsweise zweifaches Sieben herstellen, bei dem zum einen die zu kleinen Teilchen, zum anderen die zu großen Teilchen ausgesiebt werden. Unter "Teilchengröße" ist dabei etwa der maximale Teilchendurchmesser zu verstehen.

Auch das Aussieben einer Einheitsfraktion eines reinen Kugelpolymerisats ergibt eine Festigkeitszunahme und führt zu einer drastischen Abnahme des Monomergehalts, was die Verträglichkeit des Knochenzements positiv beeinflußt.

Auch bei der Ausführungsform der Erfindung, bei der dem Präpolymerisat. Fasern zugesetzt sind, können die Fasern durch anorganische oder organische Lösungsmittel oder andere Substanzen an ihrer Oberfläche vorbehandelt werden, um eine bessere Haftung zwischen Polymerkugeln, Fasern und auspolymerisierender Monomerflüssigkeit zu erreichen.

Menge und Größe des Faserersatzes des Perlpolymerisates bestimmen Misch- und Implantationsverhalten der Knochenzemente. Bei genau definierter Teilchengröße können alle Anforderungen bezüglich der Kurzzeit- als auch der Langzeitstabilität erreicht werden.

Diese Knochenzemente haben darüberhinaus den Vorteil, daß sie durch die stark verbesserten mechanischen Materialeigenschaften vorzugsweise dafür geeignet sind, durch Zusatz von resorbierbaren Füllern, wie dies in der DE—B—29 05 878 beschrieben ist, zu erheblicher Oberflächenvergrößerung in der Grenzzone beizutragen.

Gegenstand der Erfindung ist auch die Verwendung von Polyacrylsäureester- und/oder Polymethacrylsäureester oder deren Copolymerisaten in Form von Fasern mit einer Länge über 2 mm bis 40 mm, vorzugsweise bis 15 mm und einer Dicke von 50 bis 750 µm, vorzugsweise bis 500 µm, monofil oder zu Geweben vernetzt in Knochenzementen auf der Basis von Polyacrylaten und die Verwendung dieses Knochenzementes bei der Behandlung von Knochendefekten.

In einer bevorzugten Ausführungsform des erfindungsgemäßen Verfahrens wird das Mischen der Komponenten durch koaxiale Rotation beschleunigt und verbessert und das Auftreten von Monomerblasen durch Vorkomprimierung des Knochenzements während der ersten Zeit der Polymerisation mit Drücken von etwa 0,1—2 MPa, vorzugsweise 0,5—0,6 MPa verhindert, wobei die Drücke von der Viskosität des Knochenzements abhängen.

Der besondere Vorteil der erfindungsgemäßen Implantationsmaterialien liegt darin, daß durch Ersatz des Kugelpolymerisates das Material als solches nicht verändet wird, so daß lediglich eine Verbesserung der mechanischen Eigenschaften resultiert.

In vorliegender Erfindung werden faserförmige Polymerisate von Acryl- und/oder Methacrylsäureestern eingesetzt, wie sie in der Lichtleiterindustrie Verwendung finden. Auch Copolymerisate aus Acryl- und Methacrylsäureestern können verwendet werden. Bevorzugt sind Methacrylsäuremethylesterpolymerisate. Diese Materialien sind an sich bekannt und können nach bekannten Verfahren hergestellt werden. Im wesentlichen sind dies Extrudierverfahren, wobei in Fällungsbädern die Fäden gesponnen und auf Spulen aufgerollt werden Die Fäden werden in allen Dicken mit verschiedenen Polymeren und Copolymeren gefertigt und sind im Handel erhältlich. Diese Fäden werden auf Rollen als Kilometerware geliefert. Sie können anschließend in Häckselmaschinen auf die gewünschte Länge zerkleinert und in Infraschallrüttelmaschinen mit dem Polymerpulver gemischt werden.

Das Beimengen von Fasern die aus demselben Material bestehen, zum Kugelpolymerisat hat den besonderen Vorteil, daß eine optimale Benetzung der präpolymeren Bestandteile gegeben ist, wie dies bei Fremdfasern, Kohlefasern oder auch Glasfasern nicht beobachtet werden konnte. Augrund der annähernd gleichen chemischen Struktur aller Bestandteile kommt es zu einem dauerhaften und materialbeständigen Verbund, der sich weder selbst zerstört, noch inkohärent und anisotrop verhält, wie dies bei Fremdfaserverbunden nachgewiesen werden konnte.

Die negativen Eigenschaften von Fremdfaserbeimengungen konnten bei diesen Materialien ähnlicher chemischer Struktur nicht beobachtet werden.

Ein wesentlicher Vorteil der vorliegenden Erfindung ist, daß eine Materialverstärkung bei einem dennoch homogenen und in seinem mechanischen Verhalten gleichartig reagierenden Verbundmaterial erreicht wird. Auch dies konnte durch die vorliegende Erfindung überraschenderweise vorteilhaft gelöst werden. Die Oberflächenvergrößerung der Präpolymerisat-Teilchen und die Vermischung des Perlpolymerisates mit faserförmigen Polyacrylsäureester oder Polymethacrylsäureester führen nämlich zu einer vollständigen Benetzung dieser Bestandteile durch das flüssige Monomer. Fülldefekte und Lufteinschlüsse treten in weit geringerem Maße auf, als dies z.B. bei den Fremdfaserbeimischungen beobachtet werden konnte.

Es ist nun zwar in der DE—B—29 05 878 erwähnt, daß in dem dort beschriebenen Knochenzement Präpolymerisate bevorzugt werden, die in unregelmäßiger Granulatform oder als Schuppen oder als fadenförmige Zylinder vorliegen. Diese fadenförmigen Zylinder sollten eine Länge von 1—2 mm nicht überschreiten. Die Verwendung eines Gemisches aus Präpolymerisaten mit verschiedener äußerer Form, z.B. aus Kugelpolymerisat und faserförmigem Polymerisat, oder die gezielte Oberflächenvergrößerung der Präpolymerisat-Teilchen durch mechanische oder chemische Behandlung, insbesondere auch unter Auswahl einer einheitlichen Teilchengröße, wird aber in der DE—B—29 05 878 nicht gelehrt.

Es hat sich gezeigt, daß eine Fadenlänge von bis zu 2 mm keine Zunahme der Biegefestigkeit und der Torsionsfestigkeit der Knochenzemente bewirkt. Gegenüber diesen kurzen, fadenförmigen Zylindern führen Polymerkugeln zur dichteren Kugelpackung, so daß im Gegenteil eine Abnahme der mechanischen Festigkeit, insbesondere der Biegefestigkeit, durch Zusatz kurzer Fäden festgestellt werden konnte. Es wurde darüberhinaus in Versuchen festgestellt, daß ein reines Fadenpolymerisat eine drastische Verschlechterung der Mischbarkeit und der Applikationsfähigkeit der Knochenzemente nach sich zieht, und bei biologischen Untersuchungen wurde gefunden, daß das Perlpolymerisat gegenüber dem Fadenpolymerisat die besseren formbildenden Eigenschaften gegenüber den Osteoblasten, den knochenbildenden Zellen, an den Tag legt. Der mit der Erfindung angestrebte Erfolg tritt bei der Verwendung eines Gemisches aus Kugelpolymerisat und Faserzusätzen ein, wobei die Wirksamkeit der Faserbeimengungen erst bei einer Mindestlänge von über 2 mm beginnt, wobei Fasern bis zu 40 mm, vorzugsweise bis zu 15 mm Länge zugesetzt werden können. Eine optimale Materialeigenschaft wird zwischen 3 und 8 mm, insbesondere etwa 3—4 mm Faserlänge, bei einer Dicke von 100 bis 300 μm, insbesondere etwa 200 μm, erreicht. Durch Variation des Gemisches können alle möglichen Mischbarkeiten und Applikationsarten von Knochenzementen in engem Zusammenhang mit ihren Festigkeitseigenschaften festgelegt und zusammengestellt werden. Die Polymerkugeln des Kugelpolymerisates haben eine Größe von 1—140 μm, vorzugsweise von 5—60 μm, besonders bevorzugt 10—45 μm. Besonders bevorzugt ist auch bei Mischung mit Fasern eine einheitliche Teilchengröße von etwa 40 μm.

Diese Variabilität in der Herstellung von Knochenzementen hat insbesondere bei der heute geübten Praxis, bei der verschiedene Verankerungsprinzipien der Prothesenkomponenten verfolgt werden, große Vorteile.

Bei den konventionellen Gelenkersatzkomponenten werden die Prothesenkomponenten mit dem Knochenzement im Knochen verankert, d.h., die Gelenkkomponenten sind vollständig von Knochenzement umgeben und dieser verankert sie über eine große Oberfläche im knöchernen Lager. Nun wurden in den letzten Jahren auch Prothesenkomponenten entwickelt, die sich alleine, ohne Beimengung von Knochenzement, im Implantatlager verblocken. Diese sogegannten selbstverblockenden oder auch Geradschaftprothesen kommen dennoch nicht ohne Anwendung von Knochenzementen aus, denn eine Verblockung im physiologisch gekrümmten Femur beispielsweise ist nur an zwei bzw. drei Punkten möglich, so daß nach wie vor eine Rotationsinstabilität bzw. eine Instabilität in sagittaler Richtung zurückbleibt. Um dies zu verhindern, werden alle selbstverblockenden Geradschaftprothesen vorteilhaft mit Knochenzement im knöchernen Lager verblockt. Aufgrund der zumindest an drei Stellen sehr dünnen Zementscheide müssen diese Knochenzemente sehr hohen Materialanforderungen genügen. Es eignen sich hierzu vorzugsweise die Zemente, die einen hohen Acrylsäureesterfaserzusatz haben, so daß sie über eine hohe Biege- und Torsionsfestigkeit verfügen.

Falls Fasern zugesetzt werden, besteht das erfindungsgemäß eingesetzte Präpolymerisat zu 5—50, vorzugsweise 15—45 und insbesondere 20—40 Gew.-% aus fasriger Komponente. Das Gewichtsverhältnis von Präpolymerisat zu monomeren Komponeten beträgt im Knochenzement der Erfindung 50:50 bis 80:20, vorzugsweise 60:40 bis 70:30. Als Polymerisationskatalysator, Stabilisator und Beschleuniger werden die üblicherweise zu diesem Zweck in Knochenzementen eingesetzten Stoffe verwendet. Ein spezielles Beispiel für einen geeigneten Katalysator ist Dibenzoylperoxid, für einen Stabilisator Hydrochinon und für einen Beschleuniger N,N-Dimethyl-p-toluidin.

Dem Knochenzement der Erfindung kann man 5 bis 35 Gew.-%, bezogen auf die Masse des Präpolymerisates und der Monomeren, resorbierbares Tricalciumphosphat mit einer Teilchengröße zwischen 50 und 300 μm und einem verfügbaren Porenvolumen von weniger als 0,1 ml/g zusetzen. Die Art des verwendbaren Tricalciumphosphates und die dadurch erzielte Wirkung sind in der DE—B—29 05 878 beschrieben.

Zur Vermeidung von Infektionen des Implantates und des Implantatlagers, die auch durch sorgfältig aseptisches Arbeiten nicht immer verhindert werden können, die meistens sogar durch hämatogene Ausbreitung stattfinden, da das Implantat die Keime der körpereigenen Abwehr entzieht, kann dem erfindungsgemäßen Implantationsmaterial auch ein Antibiotikum beigemischt werden. Insbesondere ist eine Beimischung von Gentamycin bevorzugt, mit dem sehr gute Ergebnisse erreicht werden konnten. Die Verwendung von Gentamycin in Knochenzement ist an sich u.a. aus der DE—A—20 22 117 bekannt. Trotzdem war es nicht naheliegend, dem erfindungsgemäßen, faserverstärkten Knochenzement Gentamycin beizumischen da jede Beimengung weiterer Füllstoffe, wie der Literatur zu entnehmen ist, zu einer mechanischen Schwächung der Zemente führen muß und gerade die mechanische Festigkeit Zielsetzung dieser Faserzemente war. Es wurde aber festgestellt, daß der Zusatz von Gentamycine bis zu 6 g auf 40 g Zementpulver zu keinen wesentlichen Veränderungen der Materialeigenschaften der Knochenzemente der Erfindung führt. Dies ist durch die hohen elastischen Eigenschaften dieser Substanzen zu erklären. Insbesondere führten Gentamycinbeimengungen zwischen 1—4 Gew.-% zu keinerlei Veränderung der mechanischen Parameter.

Dem erfindungsgemäßen Knochenzement können auch andere Stoffe, wie "Bone Morphogenetic Protein" zugesetzt werden, das aus Grundsubstanz von tierischen Knochen gewonnen werden kann. Dadurch läßt sich eine Bioaktivierung der Knochenzemente erreichen, dergestalt, daß schon geringe Zusätze, homogen im Zement verteilt, zu einer beträchtlichen Beschleunigung des Knocheneinwuchses führen. Beimengungen zwischen 0,01 und 2 Gew.-% führen dabei nicht zur Veränderung der mechanischen Eigenschaften des Knochenzements.

Als weitere Zusätze kommen alle möglichen chemotherapeutisch wirksamen Substanzen, wie Zytostatika und auch andere Antibiotika und Antiseptika in Frage und z.B. auch das Hormon Calcitonin. Aus der Vielzahl der in Frage kommenden Antibiotika seien alle die erwähnt, die bei den bei der Polymerisation auftretenden Temperaturen nicht geschädigt werden: Erythromycin, Lyncomycin, Clyndamycin, Novobiocin, Vancomycin, Fusidinsäure, Rifampicin, Polymycine, Neomycin, Kanamycin oder auch Tobramycin.

Lufteinschlüsse und auch Polymerisationsblasen können durch Vorkomprimierung des Zements weitgehend vermieden werden. Die Vermischung der Knochenzemente kann dadurch verbessert werden, daß durch koaxiale Rotation eine wesentlich bessere Benetzung des Präpolymerisates erreicht wird. Wird diese Rotation vor der Vorkomprimierung durchgeführt, so kann durch geeignete Entlüftungsmittel die eingeschlossene Luft im Zementgemisch vollständig entfernt werden. Dem Mischen schließt sich dann die Vorkomprimierung mit Drücken von 0,1 bis 2 MPa, vorzugsweise 0,5 bis 0,6 MPa an. Nach der 4. Minute nach Beginn des Mischens werden die Zemente dann nach Abbau des Drucks mit langsam steigendem Kolbendruck in das Implantatlager appliziert. Die auf diese Weise vorbereiteten Knochenzemente zeigen nicht nur wesentlich bessere Materialeigenschaften, sondern weisen morphologisch auch weit weniger Blaseneinschlüsse auf. In geeigneten Fällen können sie dem industriellen Plexiglas ähnlich sein.

Durch vorliegende Erfindung konnte gezeigt werden, daß eine Mischung aus Perlpolymeren mit Faserpolymeren biologisch aktiv wirksam werden kann, da das morphologische Äquivalent des Osteoblasten, der knochenbildenden Zelle, eine 20—40 μm große Kugel darstellt. Durch teilweises Vernetzen der Polymerfasern zu Gewebsstücken mit einer Faserlänge zwischen 2 und 15 mm kann die Scherfestigkeit und Verwringungsfestigkeit der Knochenzemente ganz erheblich verbessert werden, eine Materialeigenschaft, die für die Beanspruchung des Zementköchers von ganz entscheidender Bedeutung ist.

Außer der Verstärkung mit Polymerfasern läßt sich erfindungsgemäß die mechanische Festigkeit eines Knochenzements auch dadurch erhöhen, daß man die Polymerkomponente, bevor sie dem Monomeren zugegeben wird, in einer Kugelmühle zermahlt. Auf diese Weise erhält man, wenn man den Mahlvorgang entsprechend lang durchführt, ein sehr enges Größenspektrum von kleinen Polymerkugeln mit einem Durchmesser von etwa 40 μm, da die Kugeln größerer Durchmesser durch den Mahlprozess zu Bruchstücken zertrümmert werden. Dieses Gemisch aus Polymerisat-Trümmern und Kugeln kleiner Durchmesser wird dann mit dem Monomeren vermischt und der Zement in üblicher Weise angerührt.

Besonders vorteilhaft ist es, Polymerkugeln annähernd gleicher Größe zu verwenden. Hierzu ist ein mehrfaches Aussieben der Teilchen mittels sogenannter Prüfsiebe am zweckmäßigsten. Die Differenz in den Maschenweiten der Siebe kann dabei möglichst klein gehalten werden, um den Idealfall, Polymerkugeln gleicher Durchmesser, zu erreichen. Ein Knochenzement mit dieser Polymerkomponente besitzt eine um mindestens 20 Prozent verbesserte mechanische Festigkeit gegenüber herkömmlichen Zementen.

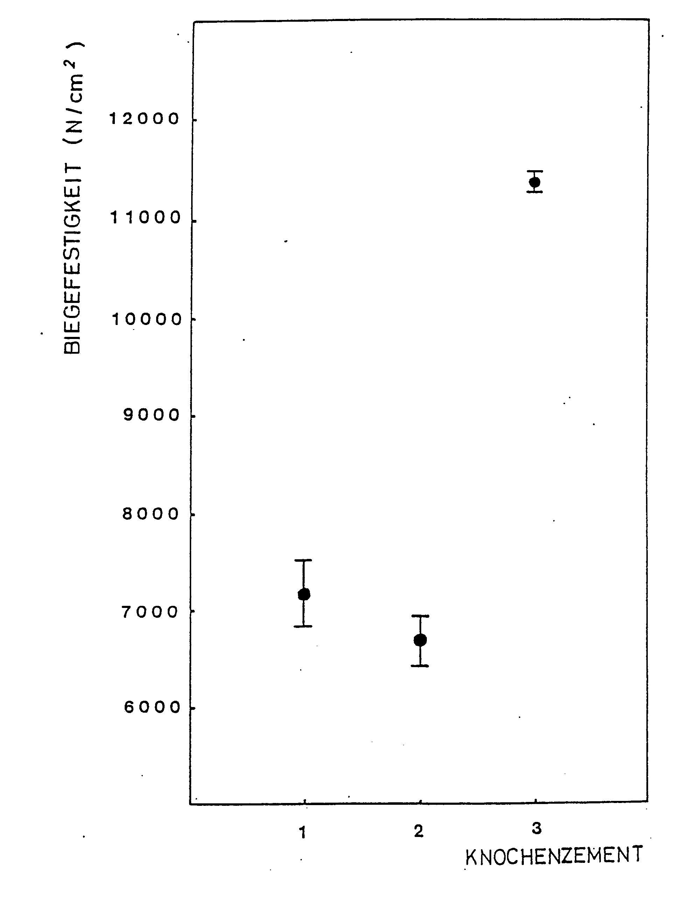

Im Biegerversuch gemäß der Figur ist die Biegefestigkeit des erfindungsgemäßen Knochenzements der unter gleichen Bedingungen ermittelten Biegefestigkeit handelsüblicher Knochenzemente gegenübergestellt (Palacos R®, Handelsname der Fa. Merck, Darmstadt, Bundesrepublik Deutschland; Sulfix-6®, Handelsname der Fa. Gebrüder Sulzer, Winterthur, Schweiz).

Zur Durchführung des Biegeversuches werden aus dem Knochenzement der Erfindung genormte Probekörper in Form eines Zylinders hergestellt.

Die Herstellung der Probekörper erfolgt unter klinischen Bedingungen, wobei sich an eine kurze Mischphase eine Komprimierungsphase von ca. 1—2 Minuten anschließt.

Die Herstellung von Probekörpern wird nachfolgend anhand von fünf Beispielen im Detail beschrieben.

### Beispiel 1

6 g eines handelsüblichen Polymethacrylsäuremethylester-Copolymerisats werden mit 4 g gehäckselten Polymethacrylsäuremethylester-Fasern mit einer Dicke von 125 µm und einer durchschnittlichen Faserlänge von 4 mm vermischt und mit einem Holzspatel in einer Kunststoff schale mit 4 ml Monomer (Methacrylsäuremethylester, Aktivator, Stabilisator) verrührt. Ist die Mischung homogen, wird sie gegebenenfalls in eine 5 ml Einwegspritze umgefüllt, der Druckstempel mit mäßig starkem Druck hineingedrückt und in eine Druckkammer gestellt. Nach Verschließen der Kammer wird mittels eines Komprimierten Gases ein Druck von 0,5 MPa während 2 Minuten angelegt. Nach Abbau des Druckes läßt man die Zementprobe in der Spritze bei Raumtemperatur aushärten.

### Beispiel 2

8 g eines handelsüblichen Polymethacrylsäuremethylester-Perlpolymerisats werden mit 2 g Polymethacrylsäuremethylester-Fasern (Dicke 125 µm, Länge 3—4 mm) vermischt und dann zu 5 ml Monomer (Methacrylsäuremethylester, Aktivator, Stabilisator) gegeben. Die Mischung wird anschließend in einer Kunststoffschale mit einem Holzspatel solange verrührt, bis eine ausreichende Homogenität erlangt wird. Dann füllt man die Mischung gegebenenfalls in eine 5 ml Einwegspritze um, drückt den Druckstempel hinein und beaufschlagt die Spritze in einer Druckkammer während 2 Minuten mit einem Druck von 0,5 MPa.

Nach Beendigung der Kompressionsphase läßt man die Zementprobe bei Zimmertemperatur aushärten.

### Beispiel 3

7 g eines handelsüblichen Polymethacrylsäuremethylester-Perlpolymerisats werden mit 3 g gehäckselten Polymethacrylsäuremethylester-Fasern mit einer Dicke von 125 µm und einer Länge von 5—15 mm vermischt und zu 4 ml Monomer (Methacrylsäuremethylester, Aktivator, Stabilisator) gegeben. Deise Mischung wird gegebenenfalls in einer 20 ml Einwegspritze bis zur Homogenität verrührt (ca. 1 Min.) und der Inhalt dann nach Erweiterung der Spritzenöffnung in eine 5 ml Einwegspritze gepreßt. Die 5 ml Einwegspritze stellt man dann in eine Druckkammer und legt einen Druck von 0,5 MPa an. Die Kompressionsphase beträgt ca. 2 Minuten. Nach Abbau des Druckes läßt man die Zementprobe in der Spritze aushärten.

### Beispiel 4

10 g eines handelsüblichen Polymethacrylsäuremethylester-Copolymerisats werden in einer Kugelmühle 2 Stunden lang mit hoher Umdrehungszahl gemahlen. Der Durchmesser der Mahlkugeln beträgt dabei 2,06 cm. Dieses Polymerisat gibt man zu 5 ml Monomer (Methacrylsäuremethylester, Aktivator, Stabilisator), das man in einer Kunststoffschale vorgelegt hat, und rührt solange, bis eine homogene Mischung entstanden ist. Gegebenenfalls wird dann die Zementmasse in eine 5 ml Eingwegspritze umgefüllt, und die Probe wird in einer Druckkammer mittels eines Komprimierten Gases bei 0,5 MPa während 2 Minuten komprimiert. Nach dieser Zeit wird der Druck wieder abgebaut und die Zementprobe in der Spritze bei Raumtemperatur ausgehärtet.

### Beispiel 5

Ca. 100 g handelsübliches Polymethacrylsäuremethylester-Copolymerisat wird auf einer Rüttelmaschine in drei übereinander angeordneten Prüfsieben mit den Maschenweiten 40 µm, 38 µm und 36 µm mehrere Stunden lang durchsiebt. Aus der Fraktion 38—40 µm werden 10 g entnommen und mit 5 ml Monomer (Methacrylsäuremethylester, Aktivator, Stabilisator) verrührt, bis die Masse homogen ist. Gegebenenfalls wird der Zement dann in eine 5 ml Einwegspritze umgefüllt und in eine Druckkammer gestellt, in der er 2 Minuten lang auf 0,5 MPa komprimiert wird. Danach wird der Druck wieder abgebaut und die Zementprobe wie üblich bei Raumtemperatur ausgehärtet.

Die beiden Verfahren gemäß den Beispielen 4 und 5, d.h. Zermahlen in der Kugelmühle und Aussieben mittels Prüfsieben, lassen sich sehr gut miteinander kombinieren, wodurch die Festigkeitsergebnisse weiter erhöht werden können.

Die nach den oben beschriebenen Verfahren herstellten Probenkörper werden mittels einer Universalprüfmaschine auf Biegebeanspruchung untersucht, wobei darauf geachtet wurde, daß die Versuchsbedingungen bei allen Messungen exakt gleich waren.

Die Ergebnisse der Biegeversuche zeigen für den erfindungsgemäßen Knochenzement Festigkeitswerte, die weit über denen der im Handel befindlichen Knochenzemente liegen. So wird mit dem in Beispiel 3 hergestellten Knochenzement ein Festigkeitswert im Biegeversuch von 11 339 N/cm$^2$ erreicht.

In der Figur ist dieser Wert als Wert Nr. 3 im Vergleich zu den Biegefestigkeiten bekannter Knochenzemente grafisch dargestellt. Die Werte Nr. 1 bzw. Nr. 2 beziehen sich auf die handelsüblichen Knochenzemente Palacos R® bzw. Sulfix-6®. Die mit den Knochenzementen der Erfindung erreichten Spitzenwerte kommen der Biegefestigkeit von reinem Plexiglas nahe.

Das Mischverhalten des beschriebenen Zementes unterscheidet sich dagegen nicht von dem handelsüblicher Knochenzemente. Die Verarbeitungs- und Härtungsphasen sind ebenfalls vergleichbar.

## Patentansprüche

1. Knochenzement, bestehend aus einem Gemisch aus teilchenförmigen Polyacrylsäureester- und/oder Polymethacrylsäureester-Präpolymerisaten, monomeren Acrylsäure- und/oder Methacrylsäurederivaten, einem Polymerisationskatalysator und gegebenenfalls einem Stabilisator und einem Aktivator, wobei die gesamte spezifische Oberfläche der Präpolymerisat-Teilchen im Knochenzement durch mechanische und/oder chemische Vorbehandlung der Teilchen vor dem Mischen vergrößert ist.

2. Knochenzement, bestehend aus einem Gemisch aus Polyacrylsäureester- und/oder Polymethacrylsäureester-Präpolymerisaten, monomeren Acrylsäure- und/oder Methacrylsäurederivaten, einem Polymerisationskatalysator und gegebenenfalls einem Stabilisator und einem Aktivator, wobei das Präpolymerisat ein Gemisch aus 5—50 Gew.-% Polyacrylsäureester- und/oder Polymethacrylsäureesterfasern mit einer Länge über 2 mm bis zu 40 mm und einer Dicke von 50—750 µm, und 50 bis 95 Gew.-% Polyacrylsäureester- und/oder Polymethacrylsäureester-Kugelpolymerisat aus Polymekugel-Teilchen ist.

3. Knochenzement nach Anspruch 2, dadurch gekennzeichnet, daß die Polyacrylsäureester- und/oder Polymethacrylsäureesterfasern monofil oder zu Geweben verflochten eingesetzt werden.

4. Knochenzement nach Anspruch 2 oder 3, dadurch gekennzeichnet, daß die Oberfläche der Polymerkugel-Teilchen und/oder der Polyacrylsäureester- und/oder Polymethacrylsäureesterfasern chemisch oder mechanisch vorbehandelt ist.

5. Knochenzement nach einem der Ansprüche 1 bis 4, dadurch gekennzeichnet, daß die kugelförmigen Teilchen durch mechanische Vorbehandlung ganz oder teilweise zu polymorphen Fragmenten zertrümmert sind.

6. Knochenzement nach einem der Ansprüche 2 bis 5, dadurch gekennzeichnet, daß die kugelförmigen Teilchen und/oder die Fasern an ihrer Oberfläche durch chemische Vorbehandlung in ihrer morphologischen Struktur verändert sind.

7. Knochenzement nach einem der Ansprüche 1 bis 6, dadurch gekennzeichnet, daß die Teilchengröße 1 bis 140 µm, vorzugsweise 5 bis 60 µm, besonders bevorzugt 10 bis 45 µm beträgt.

8. Knochenzement nach Anspruch 7, dadurch gekennzeichnet, daß die Teilchen eine möglichst einheitliche Größe aufweisen, vorzugsweise etwa 30 bis 40 µm.

9. Knochenzement nach einem der Ansprüche 1 und 8, dadurch gekennzeichnet, daß zusätzlich ein pharmazeutischer Wirkstoff enthalten ist, wie Gentamycin und/oder ein "Bone Morphogenetic Protein".

10. Knochenzement nach Anspruch 1 bis 9, dadurch gekennzeichnet, daß er zusätzlich 5 bis 35 Gew.-%, bezogen auf die Masse des Präpolymerisates und der Monomeren, resorbierbares Tricalciumphosphat mit einer Teilchengröße zwischen 50 und 300 µm und einem verfügbaren Porenvolumen von weniger als 0,1 ml/g enthält.

11. Verfahren zur Herstellung des Knochenzements nach Anspruch 1, dadurch gekennzeichnet, daß man Polyacryl- und oder Polymethacrylsäureester-Präpolymerisat aus Polymerkugel-Teilchen durch mechanische und/oder chemische Behandlung zu polymorphen Teilchen mit größerer relativer Oberfläche zerkleinert und/oder ihre Oberfläche aufrauht und danach den monomeren Acrylsäure- und/oder Methacrylsäurederivaten zusetzt und die Masse zusammen mit einem Polymerisationskatalysator und gegebenenfalls einem Stabilisator und einem Aktivator gleichmäßig vermischt.

12. Verfahren zur Herstellung des Knochenzements nach Anspruch 2, dadurch gekennzeichnet, daß man 5—50 Gew.-% Polyacrylsäureester- und/oder Polymethacrylsäureesterfasern mit einer Länge über 2 mm bis 15 mm und einer Dicke von 50—750 µm monofil oder als zu Geweben vernetzt zusammen mit 50—95 Gew.-% Polyacryl- und/oder Polymethacrylsäureester-Kugelpolymerisat aus Polymerkugel-Teilchen der Größe von 1—140 µm und/oder deren chemisch und/oder mechanisch veränderte Derivate den monomeren Acrylsäure- und/oder Methacrylsäurederivaten zusetzt und die Masse zusammen mit einem Polymerisationskatalysator und gegebenenfalls einem Stabilisator und einem Aktivator gleichmäßig vermischt.

13. Verfahren nach Anspruch 11 oder 12, dadurch gekennzeichnet, dasß man die Teilchen zuvor zu einer Fraktion einheitlicher Größe aussiebt.

14. Verwendung von Polyacrylsäureester- und/oder Polymethacrylsäureester in Form von Fasern mit einer Länge über 2 mm bis 15 mm und einer Dicke von 50—750 µm, monofil oder zu Geweben vernetzt in Knochenzementen auf der Basis von Polyacrylaten.

15. Verwendung des Knochenzements nach Anspruch 1 bis 10 bei der Behandlung von Knochendefekten.

**Revendications**

1. Ciment osseux constitué d'un mélange de prépolymères d'esters d'acide polyacrylique et/ou polyméthacrylique en forme de particules, de dérivés monomères d'acide acrylique et/ou d'acide méthacrylique, d'un catalyseur de polymérisation et éventuellement d'un agent stabilisant et d'un activeur, ciment osseux dans lequel la surface spécifique totale des particules de prépolymère dans le ciment osseux est, avant le mélange, agrandie par un prétraitement mécanique et/ou chimique des particules.

2. Ciment osseux constitué d'un mélange de prépolymères d'esters d'acide polyacrylique et/ou polyméthacrylique, de dérivés momomères d'acide acrylique et/ou d'acide méthacrylique d'un catalyseur de polymérisation et éventuellement d'un agent stabilisant et d'un activeur, ciment osseux dans lequel le prépolymère est un mélange de 5 à 50% en poids de fibres d'esters d'acide polyacrylique et/ou d'esters d'acide polyméthacrylique, qui présentent une longueur supérieure à 2 mm et allant jusqu'à 40 mm et une grosseur de 50 à 750 µm, et de 50 à 95% en poids d'un produit de polymérisation en perles d'esters d'acide polyacrylique et/ou d'esters d'acide polyméthacrylique, formé de particules de perles de polymère.

3. Ciment osseux suivant la revendication 2, caractérisé en ce que les fibres d'esters d'acide polyacrylique et/ou d'esters d'acide polyméthacrylique sont mises en oeuvre sous forme monofilamentaire ou sous forme entrelacée enn tissus.

4. Ciment osseux suivant l'une des revendications 2 et 3, caractérisé en ce que la surface des particules de perles de polymère et/ou des fibres d'esters d'acide polyacrylique et/ou d'esters d'acide polyméthacrylique est prétraitée par voie chimique ou mécanique.

5. Ciment osseux suivant l'une des revendications 1 à 4, caractérisé en ce que les particules de forme sphérique sont, par un prétraitement mécanique, totalement ou partiellement fragmentées en fragments polymorphes.

6. Ciment osseux suivant l'une des revendications 2 à 5, caractérisé en ce que les particules de forme sphérique et/ou les fibres sont modifiées sur leur surface dans leur structure morphologique par un prétraitement chimique.

7. Ciment osseux suivant l'une des revendications 1 à 6, caractérisé en ce que la grandeur des particules est de 1 à 140 µm, de préférence de 5 à 60 µm, particulièrement avantageusement de 10 à 45 µm.

8. Ciment osseux suivant la revendication 7, caractérisé en ce que les particules présentent une grandeur aussi uniforme que possible, de préférence d'environ 30 à 40 µm.

9. Ciment osseux suivant l'une des revendications 1 et 8, caractérisé en ce qu'une substance active pharmaceutique, telle que de la gentamycine et/ou une "Bone Morphogenetic Protein", est contenue en supplément.

10. Ciment osseux suivant l'une des revendications 1 à 9, caractérisé en ce qu'il contient en supplément 5 à 35% en poids, par rapport à la masse du prépolymère et du monomère, du phosphate tricalcique résorbable présentant une grandeur de particules comprise entre 50 et 300 µm et un volume de pores disponible inférieur à 0,1 ml/gr.

11. Procédé de préparation du ciment osseux suivant la revendication 1, caractérisé en ce qu'on fragmente un prépolymère d'esters d'acide polyacrylique et/ou polyméthacrylique formé de particules en perles de polymère par un traitement mécanique et/ou chimique pour former des particules polymorphes présentant une surface relative plus grande et/ou en ce qu'on rend leur surface rugueuse, et en ce qu'ensuite on ajoute le dérivé monomère d'acide acrylique et/ou d'acide méthacrylique et on mélange uniformément la masse conjointement à un catalyseur de polymérisation et éventuellement à un agent stabilisant et à un activeur.

12. Procédé de préparation du ciment osseux suivant la revendication 2, caractérisé en ce qu'aux dérivés monomères d'acide acrylique et/ou d'acide méthacrylique on ajoute 5 à 50% en poids de fibres d'esters d'acide polyacrylique et/ou d'esters d'acide polyméthacrylique qui présentent une longueur supérieure à 2 mm, allant jusqu'à 15 mm, et une grosseur de 50 à 750 µm, sous forme monofilamentaire ou sous forme réticulée en tissus, conjointement à 50—95% en poids d'un produit de polymérisation en perles d'esters d'acide polyacrylique et/ou polyméthacrylique, formé de particules en perles de polymère d'une grandeur de 1 à 140 µm et/ou leurs dérivés modifiés par voie chimique et/ou mécanique, et ce que qu'on mélange uniformément la masse conjointement à un catalyseur de polymérisation et éventuellement à un agent stabilisant et à un activeur.

13. Procédé suivant l'une des revendications 11 et 12, caractérisé en ce qu'on tamise les particules préalablement en une fraction d'une dimension uniforme.

14. Utilisation d'esters d'acide polyacrylique et/ou d'esters d'acide polyméthacrylique sous la forme de fibres présentant une longueur supérieure à 2 mm, allant jusqu'à 15 mm et une grosseur de 50 à 750 µm, sous forme monofilamentaire ou sous forme réticulée en tissus, dans des ciments osseux à base de polyacrylates.

15. Utilisation du ciment osseux suivant l'une des revendications 1 à 10 au cours du traitement des défectuosités osseuses.

**Claims**

1. Bone cement composed of a mixture of particulate polyacrylate and/or polymethacrylate prepolymers, monomeric acrylic and/or methacrylic acid derivatives, a polymerisation catalyst and, optionally, a stabiliser and an activator, wherein the entire specific surface of the prepolymer particles in the bone cement is enlarged by pretreating the particles mechanically and/or chemically before mixing them.

2. Bone cement composed of a mixture of polyacrylate and/or polymethacrylate prepolymers, monomeric acrylic and/or methacrylic acid derivatives, a polymerisation catalyst and, optionally, a stabiliser and an activator, wherein the prepolymer is a mixture of 5 to 50% by weight of polyacrylate and/or polymethacrylate fibres with a length of over 2 mm and up to 40 mm and a thickness of 50 to 750 μm, and 50 to 95% by weight of polyacrylate and/or polymethacrylate bead polymers consisting of polymer bead particles.

3. The bone cement according to claim 2, characterised in that the polyacrylate and/or polymethacrylate fibres are employed in the form of monofils or woven to textiles.

4. The bone cement according to claim 2 or 3, characterised in that the surface of the polymer bead particles and/or the polyacrylate and/or polymethacrylate fibres are pretreated chemically or mechanically.

5. The bone cement according to any of claims 1 to 4, characterised in that the bead-shaped particles have been partially or fully crushed to polymorphous fragments by prior mechanical treatment.

6. The bone cement according to any of claims 2 to 5, characterised in that the morphological structure at the surface of the bead-shaped particles and/or the fibres has been changed by prior chemical treatment.

7. The bone cement according to any of claims 1 to 6, characterised in that the particle size is 1 to 140 μm, preferably 5 to 60 μm, more preferably 10 to 45 μm.

8. The bone cement according to claim 7, characterised in that the particles are as uniform as possible in size, preferably about 30 to 40 μm.

9. The bone cement according to any of claims 1 and 8, characterised in that it also contains a pharmaceutical agent such as gentamycin and/or a "bone morphogenetic protein".

10. The bone cement according to claims 1 to 9, characterised in that it also contains 5 to 35% by weight, based on the prepolymer and monomer mass, of absorbable tricalcium phosphate having a particle size ranging from 50 to 300 μm and an available pore volume of less than 0.1 ml/g.

11. A process for preparing the bone cement according to claim 1, characterised in that polyacrylate and/or polymethacrylate prepolymers composed of polymer bead particles are broken down by mechanical and/or chemical treatment to yield polymorphous particles having a larger relative surface, and/or in that their surface is roughened, whereupon the monomeric acrylic and/or methacrylic acid derivatives are added, and in that the mass is homogenously mixed with a polymerisation catalyst and, optionally, with a stabiliser and an activator.

12. A process for preparing the bone cement according to claim 2, characterised in that 5 to 50% by weight of polyacrylate and/or polymethacrylate fibres having a length of over 2 mm and up to 15 mm and a thickness of 50 to 750 μm and in the form of monofils or woven to textiles are added together with 50 to 95% by weight of polyacrylate and/or polymethacrylate bead polymers consisting of polymer bead particles having a size of 1 to 140 μm and/or their chemically and/or mechanically treated derivatives to the monomeric acrylic and/or methacrylic acid derivatives and in that the mass is homogenously mixed with a polymerisation catalyst and, optionally, a stabiliser and an activator.

13. The process according to claim 11 or 12, characterised in that the particles are first sifted out to a fraction of uniform size.

14. The use of polyacrylate and/or polymethacrylate in the form of fibres having a length of over 2 mm and up to 15 mm and a thickness of 50 to 750 μm in the form of monofils or woven to textiles in polyacrylate-based bone cement.

15. The use of the bone cement according to claims 1 to 10 in the treatment of bone defects.